(19) Europäisches Patentamt
European Patent Office
Office européen des brevets

(11) Numéro de publication : **0 356 353 B1**

(12) # FASCICULE DE BREVET EUROPEEN

(45) Date de publication du fascicule du brevet : **11.11.92 Bulletin 92/46**

(51) Int. Cl.$^5$ : **C07C 309/14,** A01N 57/12, C07F 9/40, C07F 9/38

(21) Numéro de dépôt : **89420302.5**

(22) Date de dépôt : **10.08.89**

(54) **N-sulfonométhylglycinate, procédé de préparation, utilisation dans la préparation d'herbicides de type glyphosate.**

(30) Priorité : **18.08.88 FR 8811141**

(43) Date de publication de la demande : **28.02.90 Bulletin 90/09**

(45) Mention de la délivrance du brevet : **11.11.92 Bulletin 92/46**

(84) Etats contractants désignés : **AT BE CH DE ES FR GB GR IT LI LU NL SE**

(56) Documents cités : **DE-A- 2 606 264**

(73) Titulaire : **RHONE-POULENC AGROCHIMIE**
**14-20, rue Pierre Baizet**
**F-69009 Lyon (FR)**

(72) Inventeur : **Botannet, Bernard**
**Route du Plan Luzinay**
**F-38200 Vienne (FR)**
Inventeur : **Clavel, Jean-Louis**
**Les Rosetières Route de Champagne**
**F-69130 Ecully (FR)**
Inventeur : **Corbet, Jean-Pierre**
**7 C Chemin de Charrière Blanche**
**F-69130 Ecully (FR)**
Inventeur : **Mulhauser, Michel**
**Résidence Charrière Blanche Immeuble Frène 4**
**F-69130 Ecully (FR)**

(74) Mandataire : **Trolliet, Maurice et al**
**Rhône-Poulenc Agrochimie Département Propriété Industrielle 14-20 Rue Pierre Baizet**
**B.P. 9163**
**F-69263 Lyon Cedex 09 (FR)**

EP 0 356 353 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des composés N-sulfonométhyl glycinate, un procédé de préparation de ces composés, l'utilisation de ces composés pour la préparation d'herbicides de type glyphosate.

### Contexte technique

Le glyphosate (N-phosphonométhylglycine) et ses sels sont des herbicides à spectre large bien connus dans l'art. Ces herbicides et leurs procédés d'obtention sont par exemple décrits dans les brevets suivants : US 3,799,758 ; US 3,835,000 ; US 3,868,407 ; US 3,950,402 ; US 4,083,893 ; US 4,147,719 et EP-A-00 19384. Par ailleurs, on connait par US 4,069,048 le composé N-Sulfonométhylglycine.

### Objet de l'invention

L'invention a pour objet de proposer une voie d'accès aux herbicides de type glyphosate qui présente de nombreux avantages : économie, nombre restreint d'étapes, excellents rendements, facilité de développement à l'échelon industriel.

### L'invention

En premier lieu l'invention concerne un N-sulfonométhylglycinate de formule :

$$HO_3S - CH_2 - NH - CH_2 - COOR_1 \qquad (I)$$

formule dans laquelle :

$COOR_1$ est un groupe ester carboxylique hydrolysable.

Les groupes hydrolysables sont bien connus dans l'art. Dans le cadre de la présente invention, on entend par groupe $COOR_1$ hydrolysable tous les groupements qui peuvent être lysés en présence d'eau, éventuellement en milieu acide ou basique, pour donner l'acide -COOH et l'alcool $R_1OH$ correspondants.

Par groupe $COOR_1$ hydrolysable on entend un groupe dont le radical $R_1$ est notamment choisi parmi les radicaux suivants :

$C_1$-$C_{18}$ alkyle linéaire ou ramifié, de préférence $C_1$-$C_{12}$
$C_2$-$C_{18}$ alcényle linéaire ou ramifié, de préférence $C_2$-$C_{12}$
$C_2$-$C_{18}$ alcynyle linéaire ou ramifié, de préférence $C_2$-$C_{12}$
$C_3$-$C_{18}$ cycloalkyle linéaire ou ramifié, de préférence $C_3$-$C_{12}$
$C_6$-$C_{14}$ aryle, de préférence $C_6$-$C_{10}$
$C_7$-$C_{15}$ aralkyle linéaire ou ramifié, de préférence $C_7$-$C_{11}$,
ces radicaux étant éventuellement substitués par un ou plusieurs atomes d'halogène, radicaux $C_1$-$C_6$ alcoxy ou alkylthio, les radicaux aryle ou aralkyle pouvant, en outre, comporter 1 à 4 hétéroatomes choisis parmi l'atome d'oxygène, de soufre, d'azote (par exemple furyle, thiophènyle, pyridyle).

De préférence encore $R_1$ est choisi parmi les groupes $C_1$-$C_6$ alkyle ou $C_6$-$C_{10}$ aryle (phényle) ou $C_7$-$C_{11}$ aralkyle (benzyle, phénéthyle), lesdits radicaux étant éventuellement substitués par un ou plu -sieurs atomes d'halogène ou radicaux $C_1$-$C_6$ alkoxy.

Dans le présent exposé les composés chimiques sont désignés par leur nomenclature française mais la numérotation de la position des substituants est placée avant le nom des substituants selon la nomenclature anglosaxonne, et non pas après selon la nomenclature française.

Dans le présent exposé les formules des composés chimiques sont représentées sous forme non ioniques. Il est néammoins clair pour l'homme de métier que dans le cas des acides aminés, comme par exemple le composé de formule (I), ceux-ci peuvent exister sous forme zwitterionique.

### Procédé de préparation

Un procédé de préparation consiste à mettre en contact du formol, du dioxyde de soufre et du glycinate de formule $H_2N - CH_2 - COOR_1$.

La réaction est éffectuée, de préférence, avec les proportions molaires suivantes :

| | |
|---|---|
| glycinate | 1 mole |
| dioxyde de soufre | 0,95 mole à saturation |
| formol | 0,95 à 3 moles |

mais de préférence en proportion supérieure à celle du glycinate (1,5 à 2,5 moles).

La réaction est éffectuée généralement entre 0 et 100°C, de préférence entre 10°C et 90°C par simple

mélange des réactifs.

Le formaldéhyde est utilisé sous l'une ou l'autre forme commodément accessible. Selon une modalité la plus courante il est utilisé sous forme de solution aqueuse de concentration comprise entre 1 % et la saturation, de préférence de 30 à 40 %, en poids.

La réaction peut être effectuée en présence d'un solvant inerte ; quelquefois un tel solvant est inutile car le milieu réactionnel contient habituellement de l'eau notamment en raison de l'utilisation de formaldéhyde en solution aqueuse.

Dans le cas où le formol est utilisé sous forme non aqueuse on peut utiliser une multitude de solvants seuls ou en mélange.

Parmi les solvants aprotiques on peut citer les hydrocarbure aliphatiques saturés tels que le n-pentane, l'isopentane, le 2-méthylhexane, le 2,2,5-triméthylhexane, les hydrocarbures aromatiques tels que le benzène, le toluène, le xylène, l'éthylbenzène, les éthers aliphatiques saturés tels que le tétrahydrofuranne, l'isopropyléther, l'isopentylether, les éthers aromatiques tels que le benzyléthyléther, les cétones aliphatiques saturées ou aromatiques comme la méthyl-éthylcétone, la méthyl -isobutylcétone, l'acétophénone, les hydrocarbures halogénés saturés aliphatiques ou aromatiques comme le fluorobenzène, le 1-chloro-2-méthylpropane, le chlorure d'isobutyle, les esters aliphatiques saturés ou aromatiques comme l'isobutyrate d'isobutyle, l'acétate d'éthyle, le benzoate de méthyle. Tous ces solvants peuvent être présents seuls ou en mélange.

Parmi les solvants protiques on peut citer les alcools aliphatiques saturés ou aromatiques tels que le méthanol, l'éthanol, l'isopropanol, le phénol, les acides aliphatiques saturés ou aromatiques comme l'acide acétique, l'acide benzoïque.

Un autre procédé de préparation particulièrement avantageux et inattendu consiste à faire réagir du formol, du dioxyde de soufre, un alcool de formule $R_1OH$ et de la glycine de formule $H_2N - CH2 - COOH$.

La réaction est effectuée de préférence avec les proportions molaires suivantes :

| | |
|---|---|
| glycine : | 1 mole |
| dioxyde de soufre | 0,95 mole à saturation |
| formol | 0,95 à 3 moles |

mais de préférence en proportion supérieure à celle de la glycine (1,5 à 2,5 moles).

$R_1OH$ supérieure à 0,95 moles

mais de préférence en proportion supérieure à celle de la glycine (supérieure à 1,2 moles), avantageusement supérieure à 1,5 moles et très avantageusement supérieure à 1,8 moles.

Selon une forme avantageuse on utilisera l'alcool $R_1OH$ comme solvant, les autres solvants aprotiques cités plus haut pouvant être utilisés, éventuellement, à titre de cosolvants. De préférence, on choisira comme solvant l'éthanol.

La réaction est, par ailleurs, éffectuée dans les mêmes conditions que précédemment.

Ce dernier procédé est particulièrement préféré et par ailleur parfaitement inattendu car il conduit avec un rendement pratiquement quantitatif au N-sulfonométhyl glycinate.

L'invention a également pour objet l'utilisation des composés de formule (I) dans la préparation d'herbicides connus de type glyphosate, l'utilisation consistant à mettre en contact le composé de formule (I) avec une phosphite de formule :

$$(R_2O)_2 \underset{\overset{\|}{O}}{P}H \qquad (II)$$

$(R_2O)_2P(=O)$- étant un groupement ester phosphonique hydrolysable afin d'aboutir au composé de formule:

$$(R_2O)_2\underset{O}{P} - CH_2 - NH - CH_2 - COOR_1 \qquad (III)$$

Ces groupes hydrolysables sont bien connus de l'homme de mêtier. Par groupe $(R_2O)_2P(=O)$- hydrolysable on entend un groupe qui, sous l'action de l'eau, éventuellement en milieu acide ou basique, est lysé en alcool $R_2OH$ et acide $(O=)P(OH)_2$-.

Par groupe $(R_2O)_2P(=O)$- hydrolysable on entend un groupe dont le radical $R_2$ est notamment choisi parmi les radicaux suivants :

$C_1$-$C_{18}$ alkyle linéaire ou ramifié de préférence $C_1$-$C_{12}$

$C_2$-$C_{18}$ alcényle linéaire ou ramifié de préférence $C_2$-$C_{12}$
$C_2$-$C_{18}$ alcynyle linéaire ou ramifié de préférence $C_2$-$C_{12}$
$C_3$-$C_{18}$ cycloalkyle linéaire ou ramifié de préférence $C_3$-$C_{12}$
$C_6$-$C_{14}$ aryle linéaire ou ramifié de préférence $C_6$-$C_{10}$
$C_7$-$C_{15}$ aralkyle linéaire ou ramifié de préférence $C_7$-$C_{11}$,
ces radicaux étant éventuellement substitués par un ou plusieurs atomes d'halogène, radicaux $C_1$-$C_6$ alcoxy ou alkylthio, les radicaux aryle ou aralkyle pouvant, en outre, comporter 1 à 4 hétéroatomes choisis parmi l'atome d'oxygène, de soufre, d'azote (par exemple furyle, pyridyle, thiophènyle).

De préférence encore $R_2$ est choisi parmi les groupes $C_1$-$C_6$ alkyle ou $C_6$-$C_{10}$ aryle ou $C_7$-$C_{11}$ aralkyle, lesdits radicaux étant éventuellement substitués par un ou plusieurs atomes d'halogène ou radicaux $C_1$-$C_6$ alkoxy.

La réaction est effectuée en masse ou en solvant inerte.

Parmi les solvants aprotiques on peut citer les hydrocarbure aliphatiques saturés tels que le n-pentane, l'isopentane le 2-méthylhexane le 2,2,5-triméthylhexane, les hydrocarbures aromatiques tels que le benzène, le toluène, le xylène, l'éthylbenzène, les éthers aliphatiques saturés tels que le tétrahydrofuranne, l'isopentyléther, les éthers aromatiques tels que le benzyléthyléther, les cétones aliphatiques saturées ou aromatiques comme la méthyl-éthylcétone, la méthyl-isobutylcétone, l'acétophénone, les hydrocarbures halogénés saturés aliphatiques ou aromatiques comme le fluorobenzène, le 1-chloro-2-méthylpropane, le chlorure d'isobutyle, les esters aliphatiques saturés ou aromatiques comme l'isobutyrate d'isobutyle, l'acétate d'éthyle, le benzoate de méthyle. Tous ces solvants peuvent être présents seuls ou en mélange.

Parmi les solvants protiques on peut citer les alcools aliphatiques saturés ou aromatiques tels que le méthanol, l'isopropanol, le phénol, les acides aliphatiques saturés ou aromatiques comme l'acide acétique, l'acide benzoïque.

La température de la réaction est comprise entre 50°C et 250°C ou la température d'ebbulition du solvant et de préférence 100 à 200°C.

Bien que un large excès (3/1 à 1/3 en rapports molaires) de l'un des réactifs par rapport à l'autre soit possible, il est plus avantageux en pratique de ne pas s'écarter de plus de 20% de la stoechiometrie.

Le composé de formule (III) est un produit connu par le document EP 0135 454. Il peut être hydrolysé de manière connue pour conclure à l'herbicide N-phosphonométhyl glycine ou glyphosate.

Les exemples suivants illustrent l'invention :

Dans un ballon bicol muni d'un thermomètre à alcool sont introduits successivement 7,5 g de glycine (100 mmoles), 120 $cm^3$ d'éthanol 95 % et 19 g d'une solution aqueuse de formol à 31,5 % (200 mmoles). La suspension obtenue est saturée par du $SO_2$ jusqu'à dissolution complète (augmentation de la température jusqu'à 45°C). Après 1h30 mn d'agitation à température ambiante un début de précipitation apparaît. L'agitation est maintenue 3h supplémentaire et le précipité est filtré et lavé avec 30 $cm^3$ d'éthanol puis 30 $cm^3$ d'éther. Après sèchage 18,8 g d'acide amino méthane sulfonique du glycinate d'éthyle sont obtenus avec une pureté supérieure à 95 % (déterminée par RMN à 360 MHz) : soit un rendement de 95,5 % par rapport à la glycine de départ.

F(kofler) = 154°C.

Dans un ballon bicol muni d'un thermomètre et d'un réfrigérant sont introduits succésivement 2,46 g d'acide aminométhane sulfonique du glycinate d'éthyle (12,5 mmoles), 20 $cm^3$ de xylène et 2,07 g de diisopropylphosphite (12,5 mmoles). La suspension est chauffée 1h à 105°C et après évaporation du solvant sous vide de trompe à eau on récupère 3,81 g d'une huile orangée titrant 40 % en glyphosate protégé (N-phosphonométhyl glycinate d'isopropyle).

## Revendications

1. N-sulfonométhylglycinate de formule :

$$HO_3S - CH_2 - NH - CH_2 - COOR_1 \qquad (I)$$

$COOR_1$ étant un groupe ester carboxylique hydrolysable.

2. N-sulfonométhylglycinate selon la revendication 1, caractérisé en ce que le groupe $COOR_1$ est tel que $R_1$ est choisi parmi les radicaux suivants :
$C_1$-$C_{18}$ alkyle linéaire ou ramifié de préférence $C_1$-$C_{12}$
$C_2$-$C_{18}$ alcényle linéaire ou ramifié de préférence $C_2$-$C_{12}$
$C_2$-$C_{18}$ alcynyle linéaire ou ramifié de préférence $C_2$-$C_{12}$
$C_3$-$C_{18}$ cycloalkyle linéaire ou ramifié de préférence $C_3$-$C_{12}$
$C_6$-$C_{14}$ aryle de préférence $C_6$-$C_{10}$

$C_7$-$C_{15}$ aralkyle linéaire ou ramifié de préférence $C_7$-$C_{11}$,
ces radicaux étant éventuellement substitués par un ou plusieurs atomes d'halogène, radicaux $C_1$-$C_6$ alcoxy ou alkylthio, les radicaux aryle ou aralkyle pouvant, en outre, comporter 1 à 4 hétéroatomes choisis parmi l'atome d'oxygène, de soufre, d'azote.

**3.** N-sulfonométhylglycinate selon la revendication 2, caractérisé en ce que le groupe $COOR_1$ est tel que $R_1$ est choisi parmi les radicaux $C_1$-$C_6$ alkyle ou $C_6$-$C_{10}$ aryle ou $C_7$-$C_{11}$ aralkyle, les dits radicaux étant éventuellement substitués par un ou plusieurs atomes d'halogène ou radicaux $C_1$-$C_6$ alkoxy, $C_1$-$C_6$ alkylthio.

**4.** Procédé de préparation de N-sulfonométhylglycinate selon l'une des revendications 1 à 3, caractérisé en ce que l'on met en contact du formol, du dioxyde de soufre et du glycinate de formule :

$$H_2N - CH_2 - COOR_1$$

$COOR_1$ ayant la même signification que dans la revendication 1.

**5.** Procédé de préparation de N-sulfonométhylglycinate selon la revendication 4, caractérisé en ce que l'on met en contact les composés dans les proportions molaires suivantes :

| | |
|---|---|
| glycinate | 1 mole |
| dioxyde de soufre | 0,95 à saturation |
| formol | 0,95 à 3 moles |

**6.** Procédé de préparation de N-sulfonométhylglycinate selon l'une des revendications 1 à 3, caractérisé en ce que l'on met en contact du formol, du dioxyde de soufre, un alcool de formule $R_1OH$ et de la glycine.

**7.** Procédé de préparation de N-sulfonométhylglycinate selon la revendication 6, caractérisé en ce que l'on met en contact les composés dans les proportions molaires suivantes :

| | |
|---|---|
| glycine | 1 mole |
| dioxyde de soufre | 0,95 à saturation |
| formol | 0,95 à 3 moles |
| $R_1$ OH | supérieur à 0,95 mole |

**8.** Procédé de préparation de N-sulfonométhylglycinate selon la revendication 7, caractérisé en ce que la proportion molaire de $R_1OH$ est supérieure à 1, 2 moles et avantageusement $R_1OH$ est utilisé comme solvant.

**9.** Procédé de préparation de N-sulfonométhylglycinate selon la revendication 4 ou 6, caractérisé en ce que la réaction est effectuée entre 0 et 100°C, en solvant inerte éventuellement (ou cosolvant).

**10.** Utilisation du N-sulfonométhylglycinate de formule (I) selon l'une des revendications 1 à 3, pour la préparation d'herbicides de type glyphosate, caractérisée en ce que l'on met en contact le N-sulfonométhylglycinate de formule (I) avec un phosphonate ou une phosphite de formule :

$$(R_2O)_2P(=O)H \qquad (II)$$

$(R_2O)_2P(=O)$- étant un groupe ester phosphonique hydrolysable afin d'aboutir au composé de formule (III)

$$(R_2\ O)_2 - P(=O) - CH_2 - NH - CH_2 - COOR_1$$

ce composé pouvant être ensuite éventuellement hydrolysé de manière connue.

**11.** Utilisation selon la revendication 10, caractérisée en ce que le groupement $R_2$ est choisi parmi les groupements suivants:
$C_1$-$C_{18}$ alkyle linéaire ou ramifié de préférence $C_1$-$C_{12}$
$C_2$-$C_{18}$ alcényle linéaire ou ramifié de préférence $C_2$-$C_{12}$
$C_2$-$C_{18}$ alcynyle linéaire ou ramifié de préférence $C_2$-$C_{12}$
$C_3$-$C_{18}$ cycloalkyle linéaire ou ramifié de préférence $C_3$-$C_{12}$
$C_6$-$C_{14}$ aryle de préférence $C_6$-$C_{10}$
$C_7$-$C_{15}$ aralkyle linéaire ou ramifié de préférence $C_7$-$C_{11}$,
ces radicaux étant éventuellement substitués par un ou plusieurs atomes d'halogène, radicaux $C_1$-$C_6$ alcoxy ou alkylthio,les radicaux aryle ou aralkyle pouvant, en outre, comporter 1 à 4 hétéroatomes choisis parmi l'atome d'oxygène, de soufre, d'azote.

**12.** Utilisation selon la revendication 10,

caractérisée en ce que l'on utilise un rapport molaire (I) : (II) compris entre 1/3 et 3, avantageusement entre 0,8 et 1,2.

## Patentansprüche

1. N-Sulfomethylglycinat der Formel
$$HO_3S - CH_2 - NH - CH_2 - COOR_1 \quad (I),$$
in der $COOR_1$ eine hydrolysierbare Carbonsäureestersruppe bedeutet.

2. N-Sulfomethylglycinat nach Anspruch 1, dadurch gekennzeichnet, daß die Gruppe $COOR_1$ eine solche ist, bei der $R_1$ aus den folgenden Resten ausgewählt ist:
lineares oder verzweigtes $C_1$-$C_{18}$-, vorzugsweise $C_1$-$C_{12}$-Alkyl,
lineares oder verzweigtes $C_2$-$C_{18}$-, vorzugsweise $C_2$-$C_{12}$-Alkenyl,
lineares oder verzweigtes $C_2$-$C_{18}$-, vorzugsweise $C_2$-$C_{12}$-Alkinyl,
lineares oder verzweigtes $C_3$-$C_{18}$-, vorzugsweise $C_3$-$C_{12}$-Cycloalkyl,
lineares oder verzweigtes $C_6$-$C_{14}$-, vorzugsweise $C_6$-$C_{10}$-Aryl,
lineares oder verzweigtes $C_7$-$C_{15}$-, vorzugsweise $C_7$-$C_{11}$-Aralkyl,
wobei diese Reste gegebenfalls durch ein oder mehrere Halogenatome $C_1$-$C_6$-Alkoxy- oder Alkylthioreste substituiert sind, und die Aryl- oder Aralkylreste außerdem 1 bis 4 Heteroatome enthalten können, ausgewählt aus Sauerstoff-, Schwefel- und Stickstoffatomen.

3. N-Sulfomethylglycinat nach Anspruch 2, dadurch gekennzeichnet, daß die Gruppe $COOR_1$ eine solche ist, bei der $R_1$ ausgewählt ist unter den Resten $C_1$-$C_6$- Alkyl oder $C_6$-$C_{10}$-Phenyl oder $C_7$-$C_{11}$-Aralkyl, wobei die Reste gegebenenfalls substituiert sind durch ein oder mehrere Halogenatomen oder $C_1$-$C_6$-Alkoxy- oder $C_1$-$C_6$-Alkylthiogruppen.

4. Verfahren zur Herstellung von N-Sulfomethylglycinat nach einem der Ansprüche 1 bis 3, dadurch gekennzeichnet, daß man Formol, Schwefeldioxid und Glycinat der Formel
$$H_2N - CH_2 - COOR_1 ,$$
in der $COOR_1$ die gleiche Bedeutung wie im Anspruch 1 hat, zusammenbringt.

5. Verfahren zur Herstellurg von N-Sulfomethylglycinat nach Anspruch 4, dadurch gekennzeichnet, daß man die Verbindungen in den folgenden Molverhältnissen zusammenbringt:

| | |
|---|---|
| Glycinat | 1 mol |
| Schwefeldioxid | 0,95 mol bis zur Sättigung |
| Formol | 0,95 bis 3 mol. |

6. Verfahren zur Herstelluns von N-Sulfomethylglycinat nach einem der Ansprüche 1 bis 3, dadurch gekennzeichnet, daß man Formol, Schwefeldioxid, einen Alkohol der Formel $R_1OH$ und Glycin zusammenbringt.

7. Verfahren zur Herstellung von N-Sulfomethylglycinat nach Anspruch 6, dadurch gekennzeichnet, daß man die Verbindungen in den folgenden Molverhältnissen zusammenbringt:

| | |
|---|---|
| Glycin | 1 mol |
| Schwefeldioxid | 0,95 mol bis zur Sättigung |
| Formol | 0,95 bis 3 mol |
| $R_1OH$ | über 0,95 mol. |

8. Verfahren zur Herstellung von N-Sulfomethylglycinat nach Anspruch 7, dadurch gekennzeichnet, daß der Molanteil $R_1OH$ größer als 1, 2 ist und daß vorteilhafterweise $R_1OH$ als Lösungsmittel eingesetzt wird.

9. Verfahren zur Herstellung von N-Sulfomethylglycinat nach den Ansprüchen 4 oder 6, dadurch gekennzeichnet, daß die Reaktion zwischen 0 und 100°C, gegebenfalls in einem inerten Lösungsmittel (oder Colösungsmittel) durchgeführt wird.

10. Verwendung von N-Sulfomethylglycinat der Formel (I) nach einem der Ansprüche 1 bis 3 zur Herstellung von Herbiziden vom Glyphosphat-Typ, dadurch gekennzeichnet, daß man das N-Sulfomethylglycinat der Formel (I) mit einem Phosphonat oder einem Phosphit der Formel
$$(R_2O)_2P(=O)H \quad (II)$$

worin $(R_2O)_2P(=O)$- eine hydrolysierbare Phosphonestergruppe ist, zusammenbringt, um die Verbindung der Formel (III)

$$(R_2O)_2 - P(=O) - CH_2 - NH - CH_2 - COOR_1$$

zu erhalten, wobei diese Verbindung anschließend gegebenenfalls in an sich bekannter Weise hydrolysiert werden kann.

**11.** Verwendung nach Anspruch 10, dadurch gekennzeichnet, daß die Gruppe $R_2$ aus folgenden Gruppen ausgewählt wird:
lineares oder verzweigtes $C_1$-$C_{18}$-, vorzugsweise $C_1$-$C_{12}$-Alkyl,
lineares oder verzweigtes $C_2$-$C_{18}$-, vorzugsweise $C_2$-$C_{12}$-Alkenyl,
lineares oder verzweigtes $C_2$-$C_{18}$-, vorzugsweise $C_2$-$C_{12}$-Alkinyl,
lineares oder verzweigtes $C_3$-$C_{18}$-, vorzugsweise $C_3$-$C_{12}$-Cycloalkyl,
lineares oder verzweigtes $C_6$-$C_{14}$-, vorzugsweise $C_6$-$C_{10}$-Aryl,
lineares oder verzweigtes $C_7$-$C_{15}$-, vorzugsweise $C_7$-$C_{11}$-Aralkyl,
wobei diese Reste gegebenenfalls durch ein oder mehrere Halogenatome, $C_1$-$C_6$-Alkoxy- oder Alkylthioreste substituiert sind, und die Aryl- oder Aralkylreste außerdem 1 bis 4 Heteroatome enthalten können, ausgewählt aus Sauerstoff-, Schwefel- und Stickstoffatomen.

**12.** Verwendung nach Anspruch 10, dadurch gekennzeichnet, daß man ein Molverhältnis (I): (II) im Bereich von 1/3 bis 3, vorteilhafterweise von 0,8 bis 1,2, verwendet.

## Claims

**1.** N-sulfomethylglycinate of formula:

$$HO_3S - CH_2 - NH - CH_2 - COOR_1 \qquad (I)$$

$COOR_1$ being a hydrolysable carboxylic ester group.

**2.** N-sulfomethylglycinate according to claim 1, in which the $COOR_1$ group is such that $R_1$ is chosen from the following radicals:
linear or branched $C_1$-$C_{18}$ alkyl, preferably $C_1$-$C_{12}$
linear or branched $C_2$-$C_{18}$ alkenyl, preferably $C_2$-$C_{12}$
linear or branched $C_2$-$C_{18}$ alkynyl, preferably $C_2$-$C_{12}$
linear or branched $C_3$-$C_{18}$ cycloalkyl, preferably $C_3$-$C_{12}$
$C_6$-$C_{14}$ aryl, preferably $C_6$-$C_{10}$ linear or branched $C_7$-$C_{15}$ aralkyl, preferably $C_7$-$C_{11}$,
these radicals being optionally substituted with one or more halogen atoms or $C_1$-$C_6$ alkoxy or alkylthio radicals, the aryl or aralkyl radicals being capable, in addition, of comprising 1 to 4 hetero atoms chosen from the oxygen, sulfur and nitrogen atoms.

**3.** N-sulfomethylglycinate according to claim 2, in which the $COOR_1$ group is such that $R_1$ is chosen from the $C_1$-$C_6$ alkyl or $C_6$-$C_{10}$ aryl or $C_7$-$C_{11}$ aralkyl radicals, the said radicals being optionally substituted with one or more halogen atoms or $C_1$-$C_6$ alkoxy or $C_1$-$C_6$ alkylthio radicals.

**4.** Process for the preparation of N-sulfomethylglycinate according to one of claims 1 to 3, in which formaldehyde, sulfur dioxide and a glycinate of formula:

$$H_2N - CH_2 - COOR_1$$

are put in contact, $COOR_1$ having the same meaning as in claim 1.

**5.** Process for the preparation of N-sulfomethylglycinate according to claim 4, in which the compounds are put in contact in the following molar proportions:

| | |
|---|---|
| glycinate | 1 mole |
| sulfur dioxide | 0,95 mole to saturation |
| formaldehyde | 0,95 to 3 moles |

**6.** Process for the preparation of N-sulfomethylglycinate according to one of claims 1 to 3, in which formaldehyde, sulfur dioxide, an alcohol of formula $R_1OH$ and glycine are put in contact.

**7.** Process for the preparation of N-sulfomethylglycinate according to claim 6, in which the compounds are put in contact in the following molar proportions:

| | |
|---|---|
| glycine | 1 mole |
| sulfur dioxide | 0.95 mole to saturation |
| formaldehyde | 0.95 to 3 moles |
| $R_1OH$ | greater than 0.95 mole |

**8.** Process for the preparation of N-sulfomethylglycinate according to claim 7, in which the molar proportion of $R_1OH$ is greater than 1.2 moles, and advantageously $R_1OH$ is used as solvent.

**9.** Process for the preparation of N-sulfomethylglycinate according to claim 4 or 6, in which the reaction is carried out between 0 and 100°C, optionally in an inert solvent (or cosolvent).

**10.** Use of the N-sulfomethylglycinate of formula (I) according to one of claims 1 to 3, for the preparation of herbicides of the glyphosate type, in which the N-sulfomethylglycinate of formula (I) is put in contact with a phosphonate or a phosphite of formula:

$$(R_2O)_2P(=O)H \qquad (II)$$

$(R_2O)_2P(=0)$ - being a hydrolysable phosphonic ester group, in order to arrive at the compound of formula (III)

$$(R_2O)_2 - P(=O) - CH_2 - NH - CH_2 - COOR_1$$

this compound being capable of then being optionally hydrolysed in a known manner.

**11.** Use according to claim 10, in which the $R_2$ group is chosen from the following groups:
linear or branched $C_1$-$C_{18}$ alkyl, preferably $C_1$-$C_{12}$
linear or branched $C_2$-$C_{18}$ alkenyl, preferably $C_2$-$C_{12}$
linear or branched $C_2$-$C_{18}$ alkynyl, preferably $C_2$-$C_{12}$
linear or branched $C_3$-$C_{18}$ cycloalkyl, preferably $C_3$-$C_{12}$
$C_6$-$C_{14}$ aryl, preferably $C_6$-$C_{10}$ linear or branched $C_7$-$C_{15}$ aralkyl, preferably $C_7$-$C_{11}$,
these radicals being optionally substituted with one or more halogen atoms or $C_1$-$C_6$ alkoxy or alkylthio radicals, the aryl or aralkyl radicals being capable, in addition, of comprising 1 to 4 hetero atoms chosen from the oxygen, sulfur and nitrogen atoms.

**12.** Use according to claim 10, in which a molar ratio (I):(II) of between 1/3 and 3, and advantageously between 0.8 and 1.2, is used.